(19) Europäisches Patentamt / European Patent Office / Office européen des brevets

(11) **EP 3 610 827 B1**

(12) **EUROPEAN PATENT SPECIFICATION**

(45) Date of publication and mention
of the grant of the patent:
**16.06.2021   Bulletin 2021/24**

(51) Int Cl.:
***A61K 6/802*** *(2020.01)*

(21) Application number: **18189569.9**

(22) Date of filing: **17.08.2018**

(54) **METHOD FOR THE MANUFACTURE OF A BLANK AND BLANK**

VERFAHREN ZUR HERSTELLUNG EINES ROHLINGS SOWIE ROHLING

PROCÉDÉ DE FABRICATION D'UNE ÉBAUCHE ET ÉBAUCHE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**19.02.2020   Bulletin 2020/08**

(73) Proprietors:
• **DENTSPLY SIRONA Inc.**
  **York, PA 17401 (US)**
• **DeguDent GmbH**
  **63457 Hanau (DE)**

(72) Inventors:
• **VOELKL, Lothar**
  **63773 Goldbach (DE)**
• **FECHER, Stefan**
  **63867 Johannesberg (DE)**
• **KUTZNER, Martin**
  **63543 Neuberg (DE)**
• **OEFNER, Tanja**
  **63589 Linsengericht (DE)**
• **KOHLER, Christian**
  **63457 Hanau (DE)**
• **KRANZ, Tobias**
  **97234 Reichenberg (DE)**
• **GEBHARDT, Andreas**
  **63505 Langenselbold (DE)**
• **HAIZMANN, Martin**
  **63695 Glauburg (DE)**
• **HÖRHOLD, Heiner**
  **63654 Büdingen (DE)**
• **BURNS, Andrea**
  **63546 Hammersbach (DE)**

(74) Representative: **Dietz, Mirko**
**Sirona Dental Systems GmbH**
**Fabrikstraße 31**
**64625 Bensheim (DE)**

(56) References cited:
**DE-A1-102015 122 864     US-A1- 2016 000 538**
**US-A1- 2016 228 223     US-A1- 2017 258 563**

# Description

The invention relates to a method for the manufacture of a multilayer blank, having a lowermost layer and a topmost layer, of a ceramic material as a base material or matrix material, in particular a blank to be used for the manufacture of a dental restoration, where layers of different compositions are introduced layer-by-layer into a mold, compressed and then sintered and where individual layers contain at least one coloring oxide and preferably at least two coloring oxides.

The invention also relates to a multi-layer blank for the manufacture of a dental restoration such as a crown, partial crown, bridge, veneer, abutment, in particular anatomical abutment, comprising at least one lowermost layer and one topmost layer of ceramic material of different compositions, where the ceramic material is the base material or matrix material of the respective layer.

The subject of the invention is also a dental restoration.

A corresponding method is disclosed in DE 10 2015 122 864 A1. To achieve a continuous transition between the individual layers, an intermediate layer is formed between the individual layers and contains a mixture of the materials of the adjacent layers. A dental restoration manufactured from a corresponding blank exhibits a continuously changing translucence commencing from the bottom layer.

EP 1 900 341 B1 discloses a multi-colored molded body which consists of a number of main layers of different colors between which are intermediate layers of different colors.

A blank according to WO20151051095 A1 has at least two layers which differ both in color and translucence.

A block body of ceramic mass according to DE 10 2007 011 339 A1 has a number of layers, where in the transition region the gradient of change of the resulting optical properties is substantially constant.

Multilayer mold bodies are described in WO2010/010082 A1 and WO2015/052080 A1, where the layers have differing colors and translucencies.

A common feature of all of the multilayer blanks is that to attain different colorations / translucence properties, the coloring oxides used for this purpose decrease in quantity from the lowermost layer or bottom layer, i.e. root-side-extending layer towards the top, i.e. incisal-side-extending layer.

US 2017/0258563 A1 relates to a method for the production of a multilayer blank from ceramic material. As coloring oxide an element from the group comprising Co, Cr, Mn, Ni may be used.

US 2016/0000538 A1 refers to a method of manufacturing a zirconia block for the production of a dental prosthesis. The highest proportion of coloring oxide is in the lowermost layer.

A zirconia blank having several layers is disclosed in US 2016/0228223 A1. Said document discloses a method for the manufacture of the multilayer ceramic dental green body, where layers of different compositions are introduced layer-by-layer into a mould and pre-sintered and wherein the different layers have different chroma compositions, with increasing amounts of a chroma component through a thickness of the green body. A first chroma component may include at least one of ferric, chrome or erbium and is mainly added in the first layers. A second chroma that may include at least one of manganese oxide, neodium oxide, copper or cobalt, may be added mainly in the other (second) layers, but does not extend into the top first layer.

DE 10 2015 102 864 A1 relates to a method for the production of a blank having several ceramic layers with different proportions of coloring oxides.

The object of the present invention is to develop a method of the type mentioned at the outset such that a blank is made available from which a dental restoration can be made that can satisfy esthetic requirements and which corresponds or nearly corresponds to the natural appearance of teeth.

To achieve this aim, the method described above is developed further, as defined in claim 1, such that as a first coloring oxide as the one coloring oxide at least one oxide from the group Co, Mn, Ni, Cr is used, the proportion of which in the lowermost layer containing the first coloring oxide being lower than that in the topmost layer containing the first coloring oxide, and at least one oxide from the group Pr, Er, Fe, Ti, V, Bi, Cu, Tb is used as the second coloring oxide, the proportion of which in the lowermost layer containing the second coloring oxide is greater than that in the topmost layer.

The topmost layer is that which contains the first coloring oxide. The content of the first coloring oxide in the topmost layer is higher than in the lowermost layer.

Usually the lowermost layer is the bottom layer and the topmost layer is the top layer.

In a departure from known methods, with which multi-layer blanks are manufactured, gray-coloring metallic oxides are used, the proportion of which is lowest in the lowermost layer and highest in the topmost layer, as a result of which in comparison to the prior art it surprisingly yields a more translucent and tooth-like appearance in the region of the cutting edge, which is the same or almost the same as that of natural teeth.

For the coloring itself, at least one second coloring oxide is used in the particular layer to attain a color that corresponds to that of one tooth or a group of teeth, i.e. a bridge. The proportion of the second coloring oxide here decreases from layer to layer, commencing from the lowermost layer, i.e. the layer extending on the root side.

In particular, at least one of the metal oxide powders from the group $Al_2O_3$, $TiO_2$, zirconium oxide mixed crystal $Zr_{1-x}Me_xO_{2-(\frac{4n}{2})x}$ is used for the ceramic material, or as the ceramic material, which is the base material or matrix material of the corresponding layer, where Me is a metal that in oxide form is present as a bivalent, trivalent or tetravalent cation (n= 2, 3, 4 and $0 \leq x \leq 1$) and the tetragonal and/or the cubic phase of the zirconium oxide, where in particular yttrium oxide-stabilized zirconium dioxide is used.

$Al_2O_3$ is also emphasized as a base or matrix material of the ceramic material.

Yttrium oxide-stabilized zirconium dioxide is in particular used as ceramic material, which with respect to the individual layers can differ in its composition regardless of the added coloring oxides, for example to achieve desired strength values.

To stabilize the crystal phase in zirconium oxide at room temperature in addition to or as an alternative to $Y_2O_3$, at least one oxide from the group MgO, CaO, $CeO_2$, $ScO_3$ and $YbO_3$ can be used.

If $Y_2O_3$ is used, then it should be present in a percentage by weight of 4.5 to 13.0.

A color-intensive and naturally-appearing dental prosthesis can be made from a corresponding blank, in particular in the tooth neck region and in the dentine body. At the same time, a natural incisal region is created through the higher proportion, in comparison to the lowermost layer, of the first color oxide in the topmost layer, i.e. in the incisal region.

It was surprisingly found that, irrespective of the gray coloration of the first coloring oxide, an incisal region appears brighter and more translucent compared to a dental prosthesis in which the first coloring oxide according to the prior art decreases in proportion from the bottom or lowermost layer to the top or topmost layer.

It is in particular provided that after introduction of a layer into the mold, the surface of the layer is smoothed and a further layer is then introduced and the procedure is continued according to the number of layers.

Following introduction of the individual layers, these are then compressed as a unit.

To obtain a continuous transition between the individual layers, it is in particular provided that the smoothed surface of the last layer is structured to form elevations and depressions before introduction of a further layer.

The first coloring oxide is preferably an oxide from the group Co, Mn or mixtures thereof.

The subject of the invention is also a multi-layer blank, as defined in claim 6, for the manufacture of a dental restoration such as a crown, partial crown, bridge, veneer, abutment, in particular anatomical abutment, as an abutment having a crown, comprising at least one lowermost layer and one topmost layer of one or more ceramic materials of different compositions, where the layers contain at least one first coloring oxide, the proportion of which in the lowermost layer containing the first coloring oxide is lower than that in the topmost layer containing the first coloring oxide, wherein the first coloring oxide is at least one oxide from the group Co, Mn, Ni, Cr, in particular Co3O4 or Mn02, or a mixture thereof, and the blank contains at least one second coloring oxide from the group Pr, Bi, Er, Fe, Ti, V, Cu, Tb, the proportion of which in the lowermost layer (14) is greater than in the topmost layer.

In particular, the proportion of the first coloring oxide increases from layer to layer commencing from the lowermost layer, and the proportion of the second coloring oxide decreases from layer to layer commencing from the lowermost layer or from the layer that contains the second coloring oxide.

There is naturally no departure from the invention if, for example, the topmost layer does not contain a second coloring oxide, or if sequential layers of a multi-layer blank all have the same proportions of the coloring oxides. Irrespective thereof, the proportion of the first coloring oxide in the lowermost layer containing a first coloring oxide is lower than that in the topmost layer containing a first coloring oxide.

The invention is in particular characterized in that the proportion A1 of the first coloring oxide in the lowermost layer is 0 ppm < A1 $\leq$ 100 ppm and/or the proportion A2 of the first coloring oxide in the topmost layer is 10 ppm $\leq$ A2 $\leq$ 200 ppm, where A2 > A1.

In particular the invention provides for the proportion of the first coloring oxide to continuously change from layer to layer.

The number of layers can be selected according to the prior art. In particular, two to seven layers are provided, preferably three or four layers.

The blank of the invention may be used for the manufacture of a dental restoration such as a crown, partial crown, bridge, veneer, abutment, in particular an anatomical abutment, wherein the restoration has at least one root-side-extending lowermost layer and a incisal edge-side-extending topmost layer and preferably at least one intermediate layer extending between these, where the layers contain at least one first coloring oxide, contained in the lowermost layer in a lower proportion than in the topmost layer. The first coloring oxide is here at least one oxide from the group Co, Mn, Mi, Cr, where $Co_3O_4$ or $MnO_2$ or mixtures thereof are preferred.

The dental restoration further contains a second coloring oxide from the group Pr, Er, Fe, Ti, V, Bi, Cu, Tb, where the proportion in the lowermost layer is greater than in the topmost layer.

The dental restoration should in particular contain as ceramic material yttrium oxide ($Y_2O_3$), calcium oxide (CaO), magnesium oxide (MgO) and/or cerium oxide ($CeO_2$) and/or scandium oxide ($ScO_3$) and/or ytterbium oxide ($YbO_3$), in particular zirconium oxide to which yttrium oxide is added, where the material of the lowermost layer differs from the material of the topmost layer in color and/or proportions of crystal forms stabilized at room temperature.

Further details, advantages and characteristics of the invention are provided not just by the claims, the characteristics to be drawn from them - alone and/or in combination - but also from the following description of the preferred embodiments shown in the drawing:

> Fig. 1 shows-a schematic of a device for the manufacture of a green body,

> Fig. 2 shows-a schematic of a blank, and

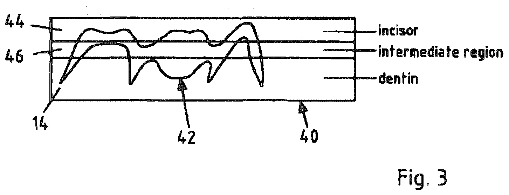

> Fig. 3 shows-a schematic of a bridge to be manufactured from a blank

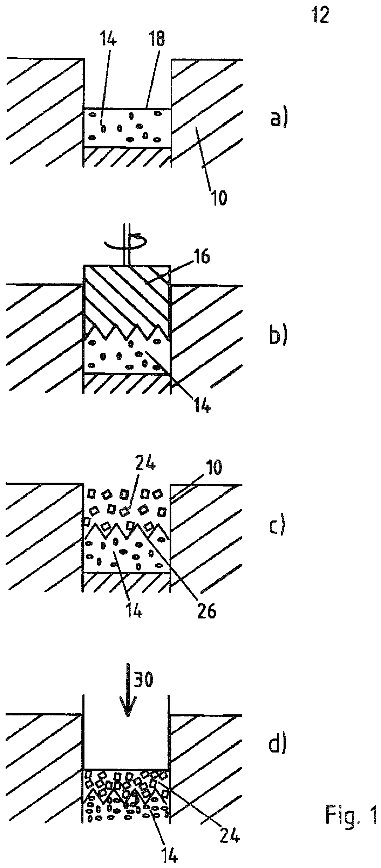

By reference to Fig. 1, it will be made clear purely in principle how a blank for a dental restoration is to be produced. Thus in accordance with Fig. 1a a first ceramic material is filled into a mold 10 of a press, in the embodiment a yttrium oxide-stabilized zirconium dioxide as the base material or matrix material, which can have the following composition in percentage by weight:

> $HfO_2 < 3.0$,
> $Al_2O_3 < 0.3$.
> Technically unavoidable impurities $< 0.2$ (such as $SiO_2$, $Na_2O$)
> $Y_2O_3$ 4.5 to 13.0
> $Co_3O_4$ as the first coloring oxide $> 0$ ppm to 200 ppm
> $Fe_2O_3$ and $Er_2O_3$ as the second coloring oxide 2,000 ppm to 8,000 ppm $ZrO_2 = 100\%$ - ($Y_2O_3$ + $Al_2O_3$ + $HfO_2$ + unavoidable impurities + coloring first oxide + coloring second oxides)

After introduction of the first layer 14, its surface is smoothed (Fig. 1a) and then structured (Fig. 1b). For this purpose, in accordance with the drawing a flat element 16 having an edge with a zig-zag geometry can be used, which penetrates the surface 18 while rotating to form a structure 26 which due to elevations and depressions, i.e. due to the element 16, consists of peaks and valleys extending in concentric circles.

The proportion of the first coloring oxide can, according to an embodiment, be in the range 3 - 8 ppm, and that of the second coloring oxide or the second coloring oxides can be between 6,500 ppm and 7,000 ppm to produce, for example, a tooth of VITA color A3.

A second ceramic material 24 is then introduced into the mold 10 (Fig. 1c), which with the exception of the first and second coloring oxides can correspond to or differ from that of the first layer 14. In particular, it is provided that the material of the second layer differs from the ceramic material of the first layer 14 in terms of yttrium oxide proportion. There is also a difference in the first coloring oxide and the second coloring oxide. The proportion of the first coloring oxide in the form of $Co_3O_4$ is greater than in the first layer 14. In particular, the proportion of $Co_3O_4$ is in the range 15 - 20 ppm. The proportion of the second coloring oxide decreases in comparison to the first layer 14 and can be in the range 5,500 to 6,000 ppm.

In addition, the proportion of yttrium oxide increases from the lowermost or bottom layer to the topmost or top layer.

With regard to the ranges of values, it is to be noted that according to the teaching of the invention, the first coloring oxide in the second layer is proportionately greater than in the first layer 14, and conversely, the second coloring oxides are proportionately lower in the second layer 24 than in the first layer 14.

The surface of the second layer 24 is then smoothed and structured so that corresponding further layers can be applied, the proportions of oxides being selected in accordance with the teaching of the invention such that the first coloring oxide increases and the proportion of the second coloring oxides decreases.

In the topmost or top layer, the proportion of the first coloring oxide, for example, which in the embodiment is $Co_3O_4$, can be between 35 ppm and 45 ppm, and the proportion of the second coloring oxides can be between 4,000 ppm and 5,000 ppm.

Once all the layers have been introduced, they are compressed in the matrix as a unit (Fig. 1d). The compressing (arrow 30) can be carried out at a pressure between, for example, 1,000 bar and 2,000 bar. Pre-sintering is then carried out at a temperature between, for example, 800 °C and 1,000 °C for a time period between, for example, 100 minutes and 150 minutes.

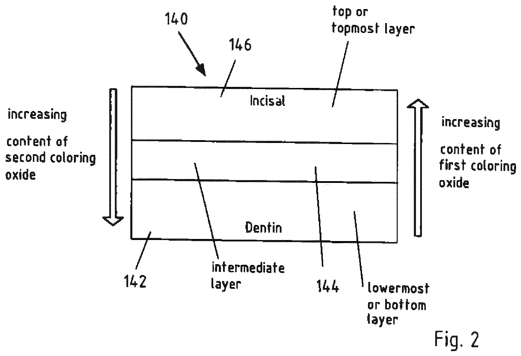

A corresponding blank 140 exhibits, as a result of the coloring oxides added to the ceramic material, a gradation in color intensity and translucence, as shown in Figure 2. In the embodiment, the blank 140 has a bottom or lowermost layer 142, an intermediate layer 144 and a top or topmost layer 146. The proportion of the first coloring oxide, such as $Co_3O_4$, increases from the bottom layer 142 in the direction of the top layer 146. By contrast, the proportion of the second coloring oxide decreases from the bottom layer (lowermost layer 142) in the direction of the top layer (topmost layer 146).

Whilst the proportion of gray cobalt oxide increases from the bottom layer in the direction of the top layer, i.e. from the dentine region to the incisal region, it was surprisingly found that a translucence can be achieved which corresponds in the cutting region of the dental prosthesis to be manufactured from the blank to that of a natural tooth, and surprisingly the gray cobalt oxide makes the incisal region brighter and more translucent.

A dental prosthesis, such as a bridge 42, can now be produced from a corresponding blank 40 in a CAD/CAM process. For this purpose, a milling program is designed such that the lower region of the bridge 42 extends in the region of the first or bottom layer 14 and the incisal region 44 extends in the top layer. The dentine region extends in the bottom region 14 and the incisal region extends in the top region with a translucence that is very close to that of a natural tooth.

If layers of zirconium oxide that differ in terms of yttrium oxide content are used, then the strength can additionally be influenced. Thus the yttrium oxide proportion in the top layer can be chosen such that the proportion of the cubic crystal phase after pre-sintering is considerably greater than that in the bottom layer 14, in which the tetragonal crystal phase should be more than 90%. In the top layer, the cubic crystal phase proportion should be between 30% and 49%. The remainder is substantially the tetragonal crystal phase.

These different crystal phases are achieved in that in the bottom layer the yttrium oxide proportion is in the range 4.5 to 7 percent by weight, and in the top layer is in the range 7 to 13.0 percent by weight, where the proportion in the first layer 14 is lower than in the top layer.

## Claims

1. A method for the manufacture of a multilayer blank (40), having a lowermost layer (14) and a topmost layer, of a ceramic material, in particular a blank to be used for the manufacture of a dental restoration, where layers (14, 24) of different compositions are introduced layer-by-layer into a mold (10), compressed and then sintered and where individual layers contain at least a first coloring oxide, wherein as the first coloring oxide at least one oxide from the group Co, Mn, Ni, Cr is used, the proportion of which in the lowermost layer (14) containing the first coloring oxide is lower than in the topmost layer containing the first coloring oxide; and where layers have at least a second coloring oxide, at least one oxide from the group Pr, Er, Fe, Ti, V, Bi, Cu, Tb is used as the second coloring oxide, the proportion of which in the lowermost layer containing the second coloring oxide is greater than that in the topmost layer.

2. The method according to claim 1, wherein after introduction of a layer (14) into the mold (10) the surface (18) of the layer is smoothed and a further layer (24) is introduced, and the procedure is continued according to the number of layers, and particularly before introduction of a further layer (24) the smoothed surface (18) of the previous layer (14) is structured to form elevations and depressions, and more particularly after introduction of all layers (14, 24) into the mold (10) the layers are compressed as a unit.

3. The method according to claim 1 or 2, wherein an oxide from the group Co, Mn or mixtures thereof is used as the first coloring oxide.

4. The method according to one of the preceding claims, wherein for the ceramic material, or as the ceramic material, at least one of the metal oxide powders from the group $Al_2O_3$, $TiO_2$, zirconium oxide mixed crystal $Zr_{1-x}Me_xO_{2-\left(\frac{4-n}{2}\right)x}$ is used, where Me is a metal that in oxide form is present as a bivalent, trivalent or tetravalent cation (n= 2, 3, 4 and $0 \leq x \leq 1$) and the tetragonal and/or cubic phase of the zirconium oxide, in particular yttrium oxide-stabilized zirconium dioxide, is used.

5. The method according to one of the preceding claims,
wherein
the lowermost layer (14) is used for a restoration (42) to be manufactured from the blank (40) for the dentine region, and the topmost layer for the incisal region.

6. A multi-layer blank (40) for the manufacture of a dental restoration (42) such as a crown, partial crown, bridge, veneer, abutment, in particular anatomical abutment, having at least one lowermost layer (14) and one topmost layer of one or more ceramic materials of different compositions, where the layers contain at least one first coloring oxide, the proportion of which in the lowermost layer containing the first coloring oxide is lower than that in the topmost layer containing the first coloring oxide; wherein the first coloring oxide is at least one oxide from the group Co, Mn, Ni, Cr, in particular $Co_3O_4$ or $MnO_2$, or a mixture thereof, and the blank contains at least one second coloring oxide from the group Pr, Bi, Er, Fe, Ti, V, Cu, Tb, the proportion of which in the lowermost layer (14) is greater than in the topmost layer.

7. The blank according to claim 6,
wherein
the proportion of the first coloring oxide increases from layer to layer, commencing from the lowermost layer (14), and/or the proportion of the second coloring oxide decreases from layer to layer, commencing from the lowermost layer (14).

8. The blank according to claim 6 or 7,
wherein
the proportion A1 of the first coloring oxide in the lowermost layer (14) is 0 ppm < A1 $\leq$ 100 ppm and/or the proportion A2 of the first coloring oxide in the topmost layer is 10 ppm $\leq$ A2 $\leq$ 200 ppm, where A2 > A1.

9. The blank according to one of the preceding claims 6 to 8,
wherein
the number of layers is between 2 and 7.

10. The blank according to one of the preceding claims 6 to 9,
wherein
the ceramic material as the base material is aluminum oxide or yttrium oxide-stabilized zirconium oxide, where the proportion of yttrium oxide is in the range 4.5 to 13.0 percent by weight and in particular the proportion increases from the lowermost layer to the topmost layer.

**Patentansprüche**

1. Verfahren zur Herstellung eines mehrschichtigen Rohlings (40), der eine unterste Schicht (14) und eine oberste Schicht aus einem Keramikmaterial aufweist, insbesondere eines Rohlings zur Herstellung einer Zahnrestauration, wobei Schichten (14, 24) verschiedener Zusammensetzung Schicht für Schicht in eine Form (10) eingebracht, komprimiert und dann gesintert werden und wobei einzelne Schichten mindestens ein erstes Farboxid enthalten, wobei

   als erstes Farboxid mindestens ein Oxid aus der Gruppe Co, Mn, Ni, Cr verwendet wird, dessen Anteil in der untersten Schicht (14), die das erste Farboxid enthält, geringer ist als in der obersten Schicht, die das erste Farboxid enthält; und wobei Schichten mindestens ein zweites Farboxid aufweisen, mindestens ein Oxid aus der Gruppe Pr, Er, Fe, Ti, V, Bi, Cu,

   Tb als zweites Farboxid verwendet wird, dessen Anteil in der untersten Schicht, die das zweite Farboxid enthält, größer ist als der in der obersten Schicht.

2. Verfahren nach Anspruch 1, wobei

   nach dem Einbringen einer Schicht (14) in die Form (10) die Oberfläche (18) der Schicht geglättet und eine weitere Schicht (24) eingebracht wird und das Verfahren entsprechend der Anzahl der Schichten fortgesetzt wird und insbesondere vor dem Einbringen einer weiteren Schicht (24) die geglättete Oberfläche (18) der vorherigen Schicht (14) so strukturiert wird, dass Erhebungen und Vertiefungen gebildet werden, und insbesondere nach dem Einbringen aller Schichten (14, 24) in die Form (10) die Schichten als Einheit komprimiert werden.

3. Verfahren nach Anspruch 1 oder 2, wobei

   ein Oxid aus der Gruppe Co, Mn oder Gemischen davon als erstes Farboxid verwendet wird.

4. Verfahren nach einem der vorstehenden Ansprüche, wobei

   für das Keramikmaterial oder als Keramikmaterial mindestens eines der Metalloxidpulver aus der Gruppe $Al_2O_3$, $TiO_2$, Zirconiumoxid-Mischkristall

   $$Zr_{1-x}Me_xO_{2-(\frac{4n}{2})x}$$ verwendet wird,

   wobei Me ein Metall ist, das in Oxidform als zweiwertiges, dreiwertiges oder vierwertiges Kation vorliegt (n = 2, 3, 4 und $0 \leq x \leq 1$) und die tetragonale und/oder kubische Phase des Zirconiumoxids, insbesondere eines Yttriumoxid-stabilisierten Zirconiumdioxids, verwendet wird.

5. Verfahren nach einem der vorstehenden Ansprüche, wobei

   die unterste Schicht (14) für eine Restauration (42), die aus dem Rohling (40) hergestellt werden soll, für den Dentinbereich und die oberste Schicht für den Inzisalbereich verwendet wird.

6. Mehrschichtrohling (40) zur Herstellung einer Zahnrestauration (42) wie einer Krone, einer Teilkrone, einer Brücke, einer Verblendung, eines Abutments, insbesondere eines anatomischen Abutments, das mindestens eine unterste Schicht (14) und eine oberste Schicht aus einem oder mehreren Keramikmaterialien unterschiedlicher Zusammensetzung aufweist, wobei die Schichten mindestens ein erstes Farboxid enthalten, dessen Anteil in der untersten Schicht, die das erste Farboxid enthält, geringer ist als der in der obersten Schicht, die das erste Farboxid enthält; wobei das erste Farboxid mindestens ein Oxid aus der Gruppe Co, Mn, Ni, Cr, insbesondere $Co_3O_4$ oder $MnO_2$ oder ein Gemisch davon ist und der Rohling mindestens ein zweites Farboxid aus der Gruppe Pr, Bi, Er, Fe, Ti, V, Cu, Tb enthält, dessen Anteil in der untersten Schicht (14) größer ist als in der obersten Schicht.

7. Rohling nach Anspruch 6, wobei

   der Anteil des ersten Farboxids von Schicht zu Schicht beginnend von der untersten Schicht (14) zunimmt und/oder der Anteil des zweiten Farboxids von Schicht zu Schicht beginnend von der untersten Schicht (14) abnimmt.

8. Rohling nach Anspruch 6 oder 7, wobei

   der Anteil A1 des ersten Farboxids in der untersten Schicht (14) 0 ppm < A1 $\leq$ 100 ppm beträgt und/oder der Anteil A2 des ersten Farboxids in der obersten Schicht 10 ppm $\leq$ A2 $\leq$ 200 ppm beträgt, wobei A2 > A1 ist.

9. Rohling nach einem der vorstehenden Ansprüche 6 bis 8, wobei

   die Anzahl der Schichten zwischen 2 und 7 liegt.

10. Rohling nach einem der vorstehenden Ansprüche 6 bis 9, wobei

    das Keramikmaterial als Grundmaterial Aluminiumoxid oder Yttriumoxid-stabilisiertes Zirconiumxoid ist, wobei der Anteil an Yttriumoxid im Bereich von 4,5 bis 13,0 Gewichtsprozent liegt und der Anteil insbesondere von der untersten Schicht zur obersten Schicht zunimmt.

## Revendications

1. Procédé de fabrication d'une ébauche multicouche (40), comportant une couche inférieure (14) et une couche supérieure, d'un matériau céramique, en particulier une ébauche à utiliser pour la fabrication d'une restauration dentaire, des couches (14, 24) de différentes compositions étant introduites couche par couche dans un moule (10), compressées, puis frittées et où les couches individuelles contenant au moins un premier oxyde colorant,
dans lequel
comme premier oxyde colorant, au moins un oxyde du groupe constitué par Co, Mn, Ni, Cr est utilisé, dont la proportion dans la couche inférieure (14) contenant le premier oxyde colorant est inférieure à celle de la couche supérieure contenant le premier oxyde colorant ; et les couches comportant au moins un deuxième oxyde colorant,
au moins un oxyde du groupe constitué par Pr, Er, Fe, Ti, V, Bi, Cu,
Tb étant utilisé comme deuxième oxyde colorant, dont la proportion dans la couche inférieure contenant le deuxième oxyde colorant est supérieure à celle de la couche supérieure.

2. Procédé selon la revendication 1,
dans lequel
après l'introduction d'une couche (14) dans le moule (10), la surface (18) de la couche est lissée et une autre couche (24) est introduite, et la procédure se poursuit en fonction du nombre de couches, et en particulier avant l'introduction d'une autre couche (24) la surface (18) lissée de la couche (14) précédente est structurée pour former des élévations et des dépressions, et plus particulièrement après l'introduction de toutes les couches (14, 24) dans le moule (10) les couches sont compressées comme une unité.

3. Procédé selon la revendication 1 ou 2,
dans lequel
un oxyde du groupe constitué par Co, Mn ou leurs mélanges est utilisé comme premier oxyde colorant.

4. Procédé selon l'une quelconque des revendications précédentes,
dans lequel
pour le matériau céramique, ou comme matériau céramique, au moins une des poudres d'oxyde métallique du groupe constitué par $Al_2O_3$, $TiO_2$, le cristal

mixte d'oxyde de zirconium $Zr_{1-x}Me_xO_{2-(\frac{4n}{2})x}$ est utilisée, où Me représente un métal, lequel, sous forme d'oxyde, est présent sous forme de cation bivalent, trivalent ou tétravalent (n = 2, 3, 4 et $0 \leq x \leq 1$) et la phase tétragonale et/ou cubique de l'oxyde de zirconium, en particulier le dioxyde de zirconium stabilisé à l'oxyde d'yttrium, est utilisée.

5. Procédé selon l'une quelconque des revendications précédentes,
dans lequel
la couche inférieure (14) est utilisée pour une restauration (42) à fabriquer à partir de l'ébauche (40) pour la zone dentinaire, et la couche supérieure pour la zone incisive.

6. Ébauche multicouche (40) destinée à la fabrication d'une restauration dentaire (42) telle qu'une couronne, une couronne partielle, un bridge, une facette, un pilier, en particulier un pilier anatomique, comportant au moins une couche inférieure (14) et une couche supérieure d'au moins un matériau céramique de différentes compositions, les couches contenant au moins un premier oxyde colorant, dont la proportion dans la couche inférieure contenant le premier oxyde colorant est inférieure à celle de la couche supérieure contenant le premier oxyde colorant ; dans laquelle le premier oxyde colorant est au moins un oxyde du groupe constitué par Co, Mn, Ni, Cr, en particulier $Co_3O_4$ ou $MnO_2$, ou un mélange de ceux-ci, et ladite ébauche contient au moins un deuxième oxyde colorant du groupe constitué par Pr, Bi, Er, Fe, Ti, V, Cu, Tb, dont la proportion dans la couche inférieure (14) est supérieure à celle dans la couche supérieure.

7. Ébauche selon la revendication 6,
dans laquelle
la proportion du premier oxyde colorant augmente de couche en couche, en commençant par la couche inférieure (14), et/ou la proportion du deuxième oxyde colorant diminue de couche en couche, en commençant par la couche inférieure (14).

8. Ébauche selon la revendication 6 ou 7,
dans laquelle
la proportion A1 du premier oxyde colorant dans la couche inférieure (14) est de 0 ppm < A1 $\leq$ 100 ppm et/ou la proportion A2 du premier oxyde colorant dans la couche supérieure est de 10 ppm $\leq$ A2 $\leq$ 200 ppm, où A2 > A1.

9. Ébauche selon l'une quelconque des revendications 6 à 8,
dans laquelle
le nombre de couches est compris entre 2 et 7.

**10.** Ébauche selon l'une quelconque des revendications 6 à 9,
dans laquelle
le matériau céramique comme matériau de base est l'oxyde d'aluminium ou l'oxyde de zirconium stabilisé à l'oxyde d'yttrium, la proportion d'oxyde d'yttrium étant comprise entre 4,5 et 13,0 pourcents en poids et en particulier la proportion augmente de la couche inférieure à la couche supérieure.

12

14   18

a)

10

16

b)

14

24   10

c)

14   26

30

d)

Fig. 1

24

14

140

146

top or
topmost layer

increasing

content of
second coloring
oxide

Incisal

Dentin

increasing

content of
first coloring
oxide

142

intermediate
layer

144

lowermost
or bottom
layer

Fig. 2

Fig. 3

incisor

intermediate region

dentin

44

46

14

42

40

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 102015122864 A1
- EP 1900341 B1
- WO 20151051095 A1
- DE 102007011339 A1
- WO 2010010082 A1
- WO 2015052080 A1
- US 20170258563 A1
- US 20160000538 A1
- US 20160228223 A1
- DE 102015102864 A1